# EUROPÄISCHE PATENTSCHRIFT

(11) **EP 0 587 107 B1**
(45) Veröffentlichungstag und Bekanntmachung des Hinweises auf die Patenterteilung: **14.08.1996**
(21) Anmeldenummer: 93114314.3
(22) Anmeldetag: 07.09.1993
(51) Int. Cl.: C07C 45/46, C07C 49/67, C07C 49/683, C07C 49/697, C07C 49/675

(54) **Verfahren zur Herstellung substituierter Indanone**
Process for the preparation of substituted indanones
Procédé de préparation d'indanones substituées

(30) Priorität: 11.09.1992 DE 4230373
(43) Veröffentlichungstag der Anmeldung: 16.03.1994
(73) Patentinhaber: HOECHST AKTIENGESELLSCHAFT, 65929 Frankfurt am Main (DE)
(72) Erfinder: Weisse, Laurent, Dr., D-61440 Oberursel (DE); Strutz, Heinz, Dr., D-61250 Usingen (DE)

(56) Entgegenhaltungen:
- EP-A- 0 199 661
- FR-A- 631 003
- US-A- 2 456 452
- JOURNAL OF THE AMERICAN CHEMICAL SOCIETY, Bd. 61, 1939, Gaston, PA, US, Seiten 1272-1281; L. F. FIESER et al: "Inter- and Intramolecular Acylations with Hydrogen Fluoride"
- JOURNAL OF THE AMERICAN CHEMICAL SOCIETY, Bd. 72, 1950, Gaston, PA, US, Seiten 3286-3287; R. T. HART et al: "Acylation - Alkylation Studies. I"

## Beschreibung

Die vorliegende Erfindung betrifft ein technisch einfaches Verfahren zur Herstellung von substituierten 1-Indanonen.

Verbindungen dieses Typs sind wichtige Zwischenprodukte bei der Herstellung von Metallocen-Komplexen, da sich 1-Indanone leicht in die entsprechenden Indene überführen lassen. Indene werden als Ligandsystem zum Aufbau von Metallocen-Komplexen verwendet (EP-A 336 128).

Weiterhin besitzen substituierte 1-Indanone technische Bedeutung als Riechstoffe (EP-A 162 465) und als wertvolle Zwischenprodukte bei der Herstellung von pharmazeutischen Produkten oder anderen bioaktiven Verbindungen (EP-A 421 759; J. Med. Chem. 25 (1990) 765).

In der Literatur sind mehrere Verfahren zur Herstellung von substituierten 1-Indanonen beschrieben.

1-Indanone, die am Sechsring Substituenten tragen, können ausgehend von den entsprechend substituierten Aromaten durch Ankondensieren des Fünfrings in 2- bis 6-stufigen Synthesen hergestellt werden (J. Org. Chem., 55 (1990) 247; Bull. Soc. Chim. Fr. 6 (1969) 1981).

Herstellungsverfahren für 1-Indanone, die am Fünfring oder an beiden Ringen Substituenten tragen sind ebenfalls bekannt (J. Org. Chem. 46 (1981) 3758; J. Org. Chem. 23 (1958) 1441).

Diese Methoden haben den Nachteil, daß sie in der Regel mehrstufig sind und nur schlechte Gesamtausbeuten an den gewünschten Produkten liefern. Viele der Synthesen sind nicht verallgemeinbar, sondern auf spezielle Derivate beschränkt. Bei anderen sind wiederum die Ausgangsmaterialien schlecht zugänglich oder sehr teuer. Gewisse Substitutionsmuster am Aromaten sind ebenfalls nach diesen Methoden nicht realisierbar. Die wenigen bekannten einstufigen Synthesen haben den Nachteil, daß sie auf spezielle Derivate beschränkt sind und schlechte Ausbeuten liefern, so daß technisch aufwendige Reinigungsoperationen der Produkte erforderlich sind. Die meisten dieser Reaktionen werden mit Hilfe von Friedel-Crafts-Katalysatoren, wie z.B. AlCl₃, durchgeführt, die im Überschuß eingesetzt werden. Diese Reaktionen erfordern technisch aufwendige Aufarbeitungsschritte, die mit einem großen Salzanfall verbunden sind.

Bekannt sind auch Herstellungsverfahren von substituierten Indanonen durch Umsetzung von Aromaten wie Xylol oder Acenaphthen mit wäßriger Methacrylsäure, Crotonsäure oder Zimtsäure in einem großen Überschuß von flüssigem Fluorwasserstoff (J. Am. Chem. Soc. 61 (1939) 1272; J. Am. Chem. Soc. 72 (1950) 3287). Die Ausbeuten liegen zwischen 62 % und 81 %. Diese Methode hat den Nachteil, daß durch vorhandenes oder gebildetes Wasser die Aktivität des Friedel-Crafts-Katalysators deutlich herabgesetzt wird. Dies führt zu niedrigen Ausbeuten und zu Korrosionsproblemen.

In EP 93 106 649.2 ist ein Verfahren beschrieben, das die Herstellung von substituierten 1-Indanonen in einem Schritt aus vergleichbar preiswerten Ausgangsverbindungen ermöglicht. Bei bestimmten Ausgangsprodukten entstehen bei dieser Methode zwei oder mehr Isomere, wobei es kaum möglich ist, bevorzugt eines dieser Isomere herzustellen. Bei stark desaktivierten Aromaten ist dieses Verfahren außerdem sehr zeitaufwendig bzw. ungeeignet.

Es bestand somit die Aufgabe, ein Verfahren zur Herstellung der obengenannten Indanone zu finden, das die aus dem Stand der Technik bekannten Nachteile vermeidet.

Überraschend wurde gefunden, daß Aromaten der nachstehenden Formel I mit käuflichen Carbonsäureanhydriden der Formel II oder Carbonsäurefluoriden der Formel III in flüssigem Fluorwasserstoff und mit Bortrifluorid schnell, nahezu quantitativ und schon bei milderen Bedingungen zu Indanonen der Formel IV/IVa reagieren. Es lassen sich außerdem einstufig bestimmte Indanone herstellen, die nach dem Stand der Technik nur durch aufwendige Synthesen zugänglich waren.

Die vorliegende Erfindung betrifft daher ein Verfahren zur Herstellung einer Verbindung der Formel IV oder deren Isomer der Formel IVa 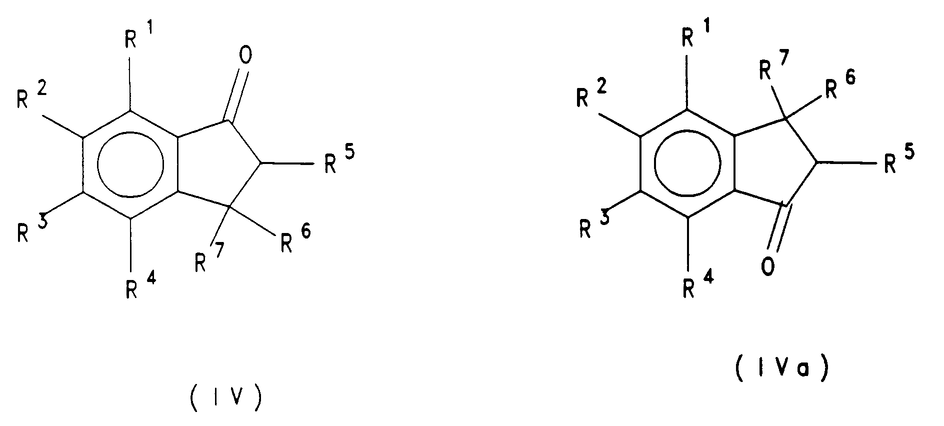
worin
R¹, R², R³, R⁴, R⁵, R⁶ und R⁷ gleich oder verschieden sind und Wasserstoff, (C₁-C₂₀)-Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest
-SiR⁸₃, wobei R⁸ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ mit den sie verbindenden Atomen einen oder mehrere substituierte oder unsubstituierte Ringe bilden, dadurch gekennzeichnet, daß man eine Verbindung der Formel I 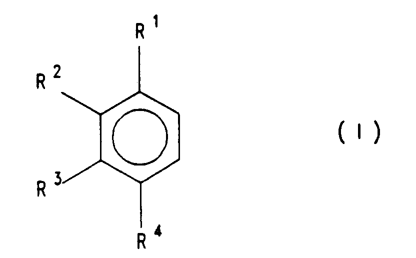
mit einer Verbindung der Formel II 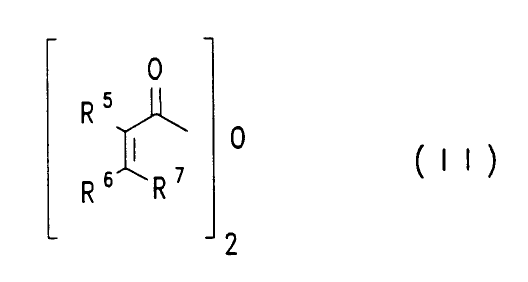
oder mit einer Verbindung der Formel (III) 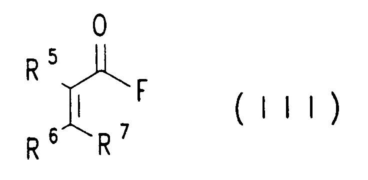
worin R¹ - R⁷ die genannten Bedeutungen besitzen, in flüssigem, wasserfreiem Fluorwasserstoff und mit Bortrifluorid umsetzt.

Dabei steht Alkyl für geradkettiges oder verzweigtes Alkyl. Halogen bedeutet Fluor, Chlor, Brom oder Jod, insbesondere Fluor oder Chlor. Beispiele für heteroaromatische Reste sind Thienyl, Furyl oder Pyridyl.

In den Formeln IV bzw. IVa gilt bevorzugt, daß R¹, R², R³, R⁴ gleich oder verschieden sind und Wasserstoff, (C₁-C₁₀)-Alkyl, (C₁-C₄)-Alkoxy, (C₂-C₆)-Alkenyl, (C₆-C₁₄)-Aryl, (C₁-C₆)-Fluoralkyl, (C₆-C₁₄)-Aryloxy oder ein Halogenatom bedeuten oder die Reste R¹ und R², R² und R³ oder R³ und R⁴ mit den sie verbindenden Atomen einen fünf- oder sechsgliedrigen Ring bilden, R⁵, R⁶ und R⁷ gleich oder verschieden sind und Wasserstoff, (C₁-C₁₀)-Alkyl oder (C₆-C₁₄)-Aryl bedeuten.

Die von benachbarten Resten R¹-R⁴ gebildeten Ringe können durch Substituenten in der Bedeutung von R¹-R⁷, beziehungsweise der dafür genannten Vorzugsbereiche, substituiert sein.

Insbesondere gilt, daß R¹, R², R³ und R⁴ gleich oder verschieden sind und Wasserstoff, (C₁-C₁₀)-Alkyl, (C₆-C₁₄)-Aryl, (C₁-C₄)-Alkoxy, oder ein Halogenatom bedeuten oder die Reste R¹ und R², R² und R³ oder R³ und R⁴ mit den sie verbindenden Atomen einen fünf- oder sechsgliedrigen, bevorzugt sechsgliedrigen gesättigten oder ungesättigten Carbocyclus bilden und R⁵, R⁶ und R⁷ gleich oder verschieden sind und für Wasserstoff, Methyl oder Phenyl stehen.

Der von benachbarten Substituenten R¹ - R⁴ gebildete gesättigte oder ungesättigte Fünf- oder Sechsring (Carbocyclus) kann zusätzlich Substituenten, bevorzugt (C₁-C₁₀)Alkyl, tragen.

Als Beispiele für Verbindungen der Formeln IV und IVa seien genannt:
6,7-Benzo-2-methylindan-1-on, 4,5-Benzo-2-methylindan-1-on,
5,7-Diisopropyl-2-methylindan-1-on, 4,6-Diisopropyl-2-methylindan-1-on,
2,5-Dimethylindan-1-on, 2,6-Dimethylindan-1-on,
5-Isobutyl-2-methylindan-1-on,
2,5,7-Trimethyl-1-indanon, 2,4,6-Trimethyl-1-indanon,
2-Methylindan-1-on,
2,4,5,6-Tetramethylindan-1-on,
5-Phenyl-2-methylindan-1-on,
8-Methyl-4,5,7,8-tetrahydrocyclopenta[e]acenaphthylen-9-on,
2-Methyl-3,9-dihydro-2H-cyclopenta[b]-fluoren-1-on,
2-Methyl-2,10-dihydro-1H-cyclopenta[a]fluoren-3-on,
16-Methyl-6,7,15,16-tetrahydrocyclopenta[a]phenanthren-17-on,
9-Methyl-5,6,9,10-tetrahydrocyclopenta[b]phenanthren-8-on,
5-Methoxy-2-methylindan-1-on,
5,6-Dimethoxy-2-methylindan-2-on.

Die Indanone können in Abhängigkeit des Substitutionsmusters am Aromaten in Form Zweier Konstitutionsisomere der Formel IV bzw. IVa anfallen. Je nach Verwendungszweck können diese in reiner Form oder als Gemisch weiter umgesetzt werden. Bei der Herstellung von Metallocen-Komplexen und bei der Verwendung der 1-Indanone als Riechstoffe kann die Isomerenmischung eingesetzt werden.

Bevorzugt werden die Indanone IV/IVa durch Umsetzung von Aromaten der Formel I mit Anhydriden der Formel II hergestellt.

Die Ausgangsverbindungen der Formel I sind im Handel erhältlich oder sie können nach literaturbekannten Methoden hergestellt werden.

Die Carbonsäurefluoride der Formel III können aus den bekannten Carbonsäurechloriden oder Carbonsäureanhydriden (Formel II) in literaturbekannter Weise durch Umsetzung mit HF hergestellt werden (vergl. z.B. Advanced Organic Chemistry, 1983, 399).

Bei der Herstellung der Verbindungen IV/IVa kann dem Fluorwasserstoff zusätzliches Lösemittel zugesetzt werden, bevorzugt wird jedoch in reinem, wasserfreiem Fluorwasserstoff gearbeitet.

Die Molverhältnisse der Verbindungen der Formel I, II oder III, und des Fluorwasserstoffs können innerhalb weiter Grenzen schwanken. Bevorzugt ist das Molverhältnis von Verbindung I : II oder III, : HF = 1 : 0,5-2,0 : 5-100; insbesondere 1 : 0,9-1,2 : 20-50.
Das heißt, es wird in einem Überschuß von Fluorwasserstoff gearbeitet.

Steht x für die Summe der Anzahl der Ether-, Keto-, Thio- bzw. Carboxygruppen in den Ausgangsverbindungen, so beträgt das Molverhältnis von Bortrifluorid zu Verbindung mit der Formel I (0,5-1,5)x : 1.

Die Reaktionstemperatur beträgt bevorzugt -30°C bis 130°C, insbesondere -10°C bis 80°C.

Die Reaktionszeiten schwanken in der Regel zwischen 10 min und 24 h, vorzugsweise zwischen 30 min und 8 h.

Bevorzugt wird die Reaktion in einem Druckbereich von 1-15.1 10⁵ Pa (1-15 atm) durchgeführt.

Bevorzugt wird eine Mischung der Verbindungen I und II (oder I und III) vorgelegt und der Fluorwasserstoff zudosiert. Auch die umgekehrte Zugabefolge ist möglich.

Nach Ende der Reaktion kann der Fluorwasserstoff abdestilliert und nahezu quantitativ ohne nennenswerte Verunreinigungen zurückgewonnen werden. Auch die Rückgewinnung von Bortrifluorid ist grundsätzlich möglich.

Die Indanone der Formel IV bzw. IVa können durch Waschen mit Na₂CO₃-, NaHCO₃- oder KOH-Lösung und Wasser von Säureanteilen befreit und mit den üblichen Trockenmitteln wie Na₂SO₄, MgSO₄ oder Molekularsieben getrocknet werden. Da die Umsetzungen in der Regel nahezu quantitativ sind, kann in den meisten Fällen auf eine weitere Reinigung verzichtet werden. Oft empfiehlt sich jedoch eine Filtration über Kieselgel, Aluminiumoxid oder Filterhilfsmitteln, wie z.B. Celite. Falls notwendig, kann die weitere Reinigung durch Destillation, Säulenchromatographie oder Kristallisation erfolgen. Falls erforderlich können die Konstitutionsisomeren III und IIIa durch Säulenchromatographie an Kieselgel oder Aluminiumoxid voneinander getrennt werden.

Das erfindungsgemäße Verfahren zeichnet sich besonders dadurch aus, daß unterschiedlich substituierte 1-Indanone in einer einfachen und kurzen Synthese (Einstufenprozeß) und in nahezu quantitativer Ausbeute erhalten werden können. Dabei können die Raumzeitausbeuten durch die Verwendung von Bortrifluorid gegenüber dem Stand der Technik erheblich verbessert werden. Ein weiterer Vorteil dieser Methode ist es, durch geringfügiges Verändern der Reaktionsbedingungen (z.B. Temperatur), die Produktselektivität optimieren zu können. Ein besonderer Vorteil ist, daß man mit dem erfindungsgemäßen Verfahren auch elektronenarme Aromaten wie z.B. Fluorbenzol zum entsprechenden Indanon umsetzen kann, was nach bisherigem Stand der Technik nicht möglich war.

Bevorzugt werden die Indanone IV/IVa zur Herstellung von Metallocenen (vgl. z.B. EP-A 336 128) oder als Riechstoffe (EP-A 162 465) verwendet.

Die nachfolgenden Beispiele dienen der näheren Erläuterung der Erfindung.

### Beispiel A

### 6,7-Benzo-2-methylindan-1-on (1) und 4,5-Benzo-2-methylindan-1-on (1a)

12,6 g (98 mmol) Naphthalin, 15,8 g (103 mmol) Methacrylsäureanhydrid und 100 g (5 mol) Fluorwasserstoff wurden in einem 250 ml Edelstahlautoklaven mit 14 g Bortrifluorid versetzt und 1 h bei -10°C umgesetzt. Anschließend wird die Reaktionsmischung auf 1 Kilo Eis gegeben und die Lösung mit verdünnter KOH neutralisiert. Nach mehrmaligem Waschen der wäßrigen Phase mit Essigester wurden die abgetrennten und getrockneten organischen Phasen im Vakuum vom Lösemittel befreit. Dabei erhielt man 18,7 g (95 % Ausbeute) eines Gemisches aus (1) und (1a) mit einer Selektivität von 81 bzw. 19 %.

### Vergleichsbeispiel zu A

12,6 g (98 mmol) Naphthalin und 15,8 g (103 mmol) Methacrylsäureanhydrid wurden in 100 g (5 mol) HF 18 h bei 50°C umgesetzt. Die Aufarbeitung erfolgte analog Beispiel A. Dabei wurden 19 g (97 % Ausbeute) Produkt erhalten. Die Selektivität betrug 58 % der Verbindung (1), bzw. 39 % der Verbindung (1a).

### Beispiel B

### 5-Fluor-2-methyl-indan-1-on (2)

9,6 g (100 mmol) Fluorbenzol, 15,8 g (103 mmol) Methacrylsäureanhydrid und 100 g (5 mol) HF wurden analog Beispiel A mit 14 g (206 mmol) Bortrifluorid versetzt und 3 h bei 50°C gerührt. Die Aufarbeitung erfolgte analog Beispiel A. Dabei erhielt man 16 g (97 % Ausbeute) einer fast farblosen Flüssigkeit. Die Selektivität zu (2) betrug 82 %.

### Vergleichsbeispiel zu B

9,6 g (100 mmol) Fluorbenzol, 16 g (104 mmol) Methacrylsäureanhydrid und 100 g (5 mol) HF wurden 18 h bei 70°C umgesetzt. Die Aufarbeitung erfolgte analog Beispiel A. Dabei wurde kein 5-Fluor-2-methylindan-1-on isoliert.

### Beispiel C

### 2-Methyl-5-phenylindan-1-on (3)

15,4 g (100 mmol) Biphenyl, 16 g (104 mmol) Methacrylsäureanhydrid, 100 g (5 mol) HF und 14 g (206 mmol) BF₃ wurden analog Beispiel A 2 h bei 50°C umgesetzt und aufgearbeitet. Dabei wurden 22 g (99 % Ausbeute) Produkt isoliert. Die Selektivität zu (3) betrug 94 %.

### Vergleichsbeispiel zu C

15,4 g (100 mmol) Biphenyl und 16 g (104 mmol) Methacrylsäureanhydrid wurden mit 100 g (5 mol) HF 60 h bei 70°C umgesetzt. Nach der analog A durchgeführten Aufarbeitung wurden 22,2 g (100 % Ausbeute) Produkt gewonnen. Die Reinheit betrug 92 %.

## Patentansprüche

1. Verfahren zur Herstellung einer Verbindung der Formel IV oder deren Isomer der Formel IVa 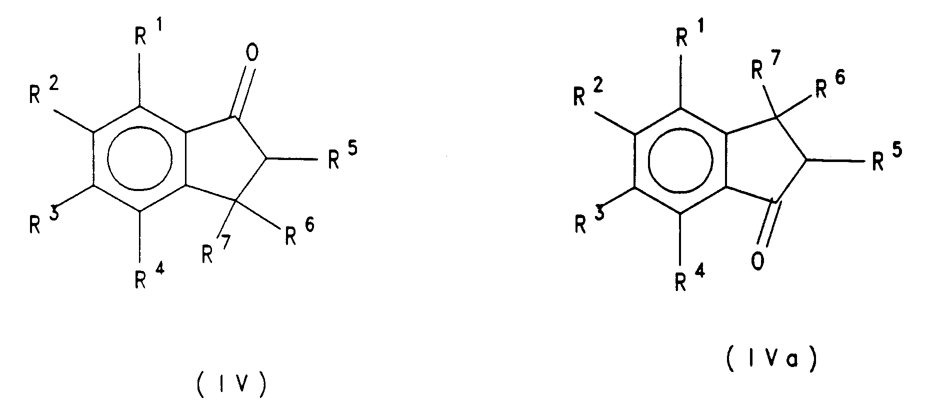 worin
R¹, R², R³, R⁴, R⁵, R⁶ und R⁷ gleich oder verschieden sind und Wasserstoff, (C₁-C₂₀)-Alkyl, (C₆-C₁₄)Aryl, (C₁-C₁₀)Alkoxy, (C₂-C₁₀)Alkenyl, (C₇-C₂₀)Arylalkyl, (C₇-C₂₀)Alkylaryl, (C₆-C₁₀)Aryloxy, (C₁-C₁₀)Fluoralkyl, (C₆-C₁₀)Halogenaryl, (C₂-C₁₀)Alkinyl, einen Rest
-SiR⁸₃, wobei R⁸ für (C₁-C₁₀)Alkyl steht, ein Halogenatom oder einen heteroaromatischen Rest mit 5 oder 6 Ringgliedern, der ein oder mehrere Heteroatome enthalten kann, bedeuten, oder benachbarte Reste R¹-R⁴ mit den sie verbindenden Atomen einen oder mehrere substituierte oder unsubstituierte Ringe bilden, dadurch gekennzeichnet, daß man eine Verbindung der Formel I 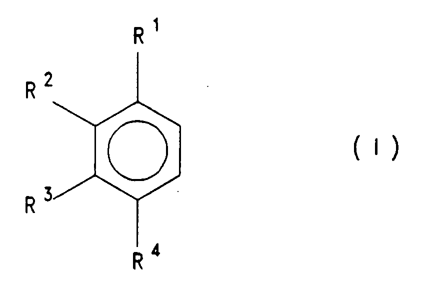 mit einer Verbindung der Formel II 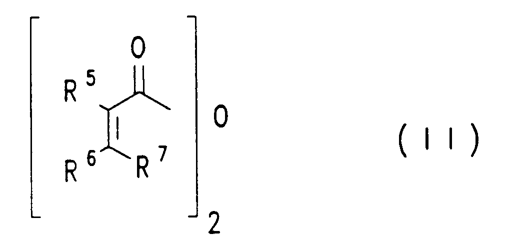 oder mit einer Verbindung der Formel (III) 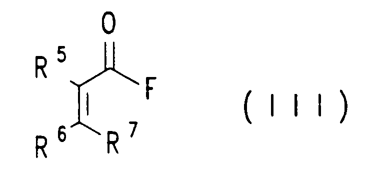 worin R¹ - R⁷ die genannten Bedeutungen besitzen, in flüssigem, wasserfreiem Fluorwasserstoff und mit Bortrifluorid umsetzt.

2. Verfahren gemäß Anspruch 1, dadurch gekennzeichnet, daß in den Formeln IV und IVa R¹, R², R³, R⁴ gleich oder verschieden sind und Wasserstoff, (C₁-C₁₀)Alkyl, (C₁-C₄)Alkoxy, (C₂-C₆)Alkenyl, (C₆-C₁₄)Aryl, (C₁-C₆)Fluoralkyl, (C₆-C₁₄)-Aryloxy oder ein Halogenatom bedeuten oder die Reste R¹ und R², R² und R³ oder R³ und R⁴ mit den sie verbindenden Atomen einen fünf- oder sechsgliedrigen Ring bilden, R⁵, R⁶ und R⁷ gleich oder verschieden sind und Wasserstoff, (C₁-C₁₀)Alkyl oder (C₆-C₁₄)Aryl bedeuten.

3. Verfahren gemäß Anspruch 1 oder 2, dadurch gekennzeichnet, daß in den Formeln IV und IVa R¹, R², R³ und R⁴ gleich oder verschieden sind und Wasserstoff, (C₁-C₁₀)Alkyl, (C₆-C₁₄)Aryl, (C₁-C₄)Alkoxy oder ein Halogenatom bedeuten oder die Reste R¹ und R², R² und R³ oder R³ und R⁴ mit den sie verbindenden Atomen einen sechsgliedrigen, gesättigten oder ungesättigten Carbocyclus bilden und R⁵, R⁶ und R⁷ gleich oder verschieden sind und für Wasserstoff, Methyl oder Phenyl stehen.

4. Verfahren gemäß einem oder mehreren der Ansprüche 1 bis 3, dadurch gekennzeichnet, daß das molare Verhältnis von Verbindung I : Verbindung II oder Verbindung III : Fluorwasserstoff 1 : 0,5-2,0 : 5-100 beträgt.

5. Verfahren gemäß einem oder mehreren der Ansprüche 1 bis 4, dadurch gekennzeichnet, daß das molare Verhältnis von Bortrifluorid: Verbindung I (0,5-1,5)x : 1 beträgt, worin x für die Summe der Anzahl der Ether-, Keto-, Thio- bzw. Carboxygruppen in den Ausgangsverbindungen steht.

## Claims

1. A process for the preparation of a compound of the formula IV or the isomer thereof of the formula IVa 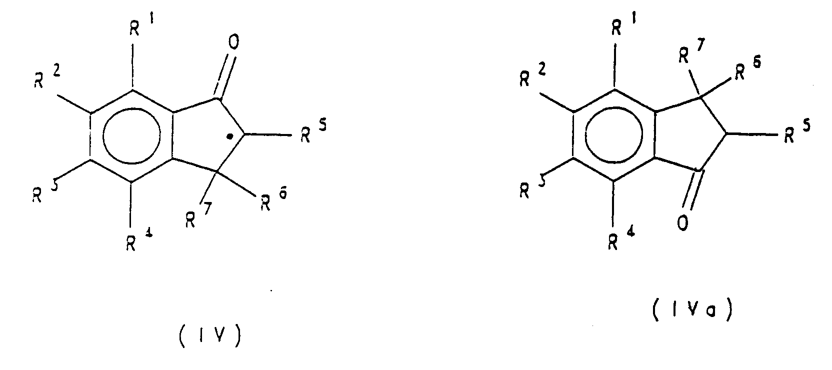 in which
R¹, R², R³, R⁴, R⁵, R⁶ and R⁷ are identical or different and are hydrogen, (C₁-C₂₀)-alkyl, (C₆-C₁₄)aryl, (C₁-C₁₀)alkoxy, (C₂-C₁₀)alkenyl, (C₇-C₂₀)arylalkyl, (C₇-C₂₀)alkylaryl, (C₆-C₁₀)aryloxy, (C₁-C₁₀)fluoroalkyl, (C₆-C₁₀)haloaryl, (C₂-C₁₀)alkynyl, an -SiR⁸₃ radical where R⁸ is (C₁-C₁₀)alkyl, or are a halogen atom or a heteroaromatic radical having 5 or 6 ring members which may contain one or more heteroatoms, or adjacent radicals R¹-R⁴, together with the atoms connecting them, form one or more substituted or unsubstituted rings, which comprises reacting a compound of the formula I 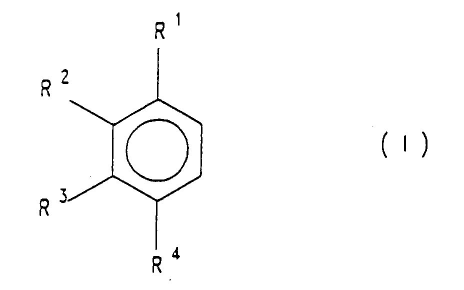 with a compound of the formula II 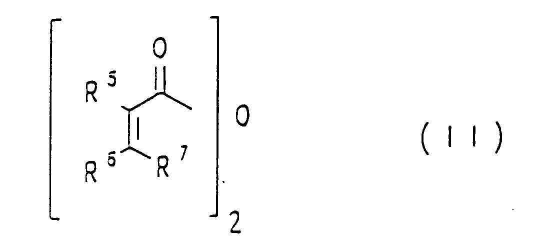 or with a compound of the formula (III) 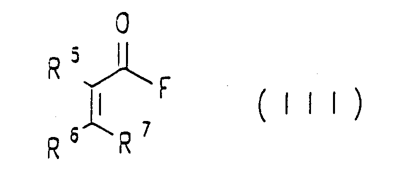 in which R¹-R⁷ are as defined above, in liquid, anhydrous hydrogen fluoride and with boron trifluoride.

2. The process as claimed in claim 1, wherein, in the formulae IV and IVa, R¹, R², R³ and R⁴ are identical or different and are hydrogen, (C₁-C₁₀)alkyl, (C₁-C₄)alkoxy, (C₂-C₆)alkenyl, (C₆-C₁₄)aryl, (C₁-C₆)fluoroalkyl, (C₆-C₁₄)aryloxy or a halogen atom, or the radicals R¹ and R², R² and R³ or R³ and R⁴, together with the atoms connecting them, form a five- or six-membered ring, and R⁵, R⁶ and R⁷ are hydrogen, (C₁-C₁₀)alkyl or (C₆-C₁₄)aryl.

3. The process as claimed in claim 1 or 2, wherein, in the formulae IV and IVa, R¹, R², R³ and R⁴ are identical or different and are hydrogen, (C₁-C₁₀)alkyl, (C₆-C₁₄)aryl, (C₁-C₄)alkoxy or a halogen atom, or the radicals R¹ and R², R² and R³ or R³ and R⁴, together with the atoms connecting them, form a six-membered, saturated or unsaturated carbocyclic ring, and R⁵, R⁶ and R⁷ are identical or different and are hydrogen, methyl or phenyl.

4. The process as claimed in one or more of claims 1 to 3, wherein the compound I : compound II or compound III : hydrogen fluoride molar ratio is from 1 : 0.5-2.0 : 5-100.

5. The process as claimed in one or more of claims 1 to 4, wherein the boron trifluoride: compound I molar ratio is from (0.5-1.5)x : 1, in which x is the total number of ether, keto, thio or carboxyl groups in the starting compounds.

## Revendications

1. Procédé pour la préparation d'un composé de formule IV ou de son isomère de formule IVa 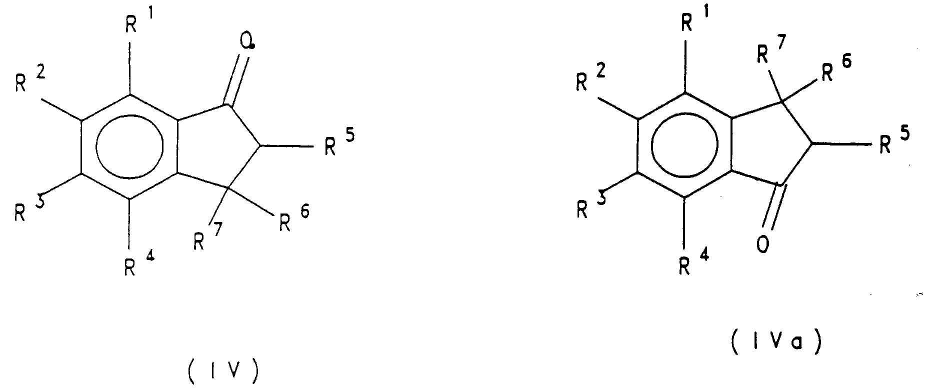 où,
R¹, R², R³, R⁴, R⁵, R⁶ et R⁷ sont identiques ou différents et représentent l'hydrogène, alkyle en C₁-C₂₀, aryle en C₆-C₁₄, alcoxy en C₁-C₁₀, alcényle en C₂-C₁₀, arylalkyle en C₇-C₂₀, alkylaryle en C₇-C₂₀, aryloxy en C₆-C₁₀, fluoralkyle en C₁-C₁₀, halogénoaryle en C₆-C₁₀, alcynyle en C₆-C₁₀, un radical -SiR⁸₃
R⁸ étant alkyle en C₁-C₁₀, un atome d'halogène ou un radical hétéroaromatique avec 5 ou 6 chaînons, qui peuvent contenir un ou plusieurs hétéro-atomes, ou les radicaux R¹ à R⁴ voisins forment avec les atomes qui les relient un ou plusieurs cycles substitués ou non substitués, caractérisés en ce qu'on fait réagir un composé de formule I 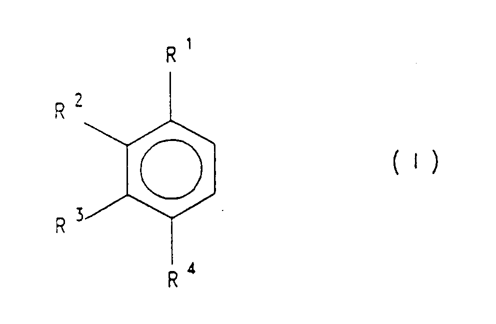 avec un composé de formule II 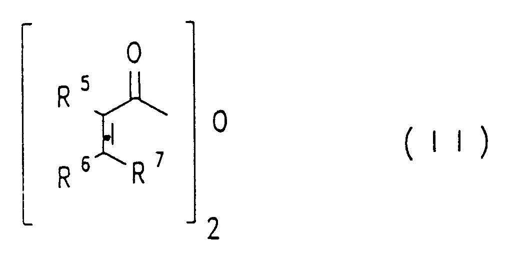 ou avec un composé de formule III 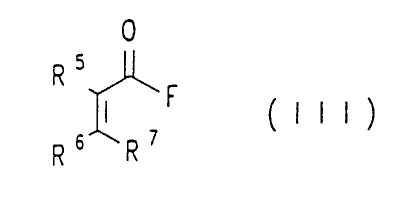 où R¹ à R⁷ ont les significations précitées, dans du fluorure d'hydrogène anhydre liquide et avec du trifluorure de bore.

2. Procédé selon la revendication 1 caractérisé en ce que dans les formules IV et IVa, R¹, R², R³, R⁴ sont identiques ou différents et représentent l'hydrogène, alkyle en C₁-C₁₀, alcoxy en C₁-C₄, alcényle en C₂-C₆, aryle en C₆-C₁₄, fluoroalkyle en C₁-C₆, aryloxy en C₆-C₁₄ ou un atome d'halogène et les radicaux R¹ et R², R² et R³ ou R³ et R⁴, avec les atomes qui les relient forment un cycle à 5 ou 6 chaînons, R⁵, R⁶ et R⁷ sont identiques ou différents et représentent l'hydrogène, alkyle en C₁-C₁₀ ou aryle en C₆-C₁₄.

3. Procédé selon la revendication 1 ou 2, caractérisé en dans les formules IV et IVa R¹, R², R³ et R⁴ sont identiques ou différents et représentent l'hydrogène, alkyle en C₁-C₁₀, aryle en C₆-C₁₄, alcoxy en C₁-C₄ ou un atome d'halogène ou les radicaux R¹ et R², R² et R³ ou R³ et R⁴ avec les atomes qui les relient forment un carbocycle à 6 chaînons, saturé ou insaturé et R⁵, R⁶ et R⁷ sont identiques ou différents et représentent l'hydrogène, méthyle ou phényle.

4. Procédé selon une ou plusieurs des revendications 1 à 3, caractérisé en ce que le rapport molaire des composés I : composé II ou composé III : fluorure d'hydrogène est égal à 1 : 0,5 à 2,0 : 5 à 100.

5. Procédé selon une ou plusieurs des revendications 1 à 4, caractérisé en ce que le rapport molaire de trifluorure de bore : composé I (0,5-1,5)x : 1, où x représente la somme du nombre des groupes éther, céto, thio, respectivement carboxy dans les composés de départ.
